# EUROPEAN PATENT APPLICATION

(11) **EP 2 705 790 A1**
(43) Date of publication of application: **12.03.2014**
(21) Application number: 13183226.3
(22) Date of filing: 05.09.2013
(51) Int. Cl.: A61B 5/11, G08B 21/04

(54) **A monitoring system for a resting surface**

(30) Priority: 05.09.2012 ES 201230923 U
(71) Applicant: Corujo Bolaños, Gabriel, 35412 Las Palmas (ES)
(72) Inventor: Corujo Bolaños, Gabriel, 35412 Las Palmas (ES)
(74) Representative: Isern-Jara, Nuria

(57) **Abstract**

RESTING SURFICE MONITORING SYSTEM, composed by different parts, first of them is the resting surface wich integrates the distribution of sensor elements that form the invention, while another of the defined part is the one in charge of the managing of the logical data gathering from the elements, composed of an electronic circuit in charge of the monitoring and managing of the variations produced by the sensors and allowing the information sending through different communication technologies, such as guided or not guided communication systems, with a data base thata allows the administration and managing of the storaged data.

## Description

### OBJECT OF THE INVENTION

The invention which is presented in this specification report refers to the deployments of elements that capture variations of different parameters, integrated in a surface protector. The data is read through the variation data gathering elements, their interpretation, storage and finally the interaction of the users with the different stored data.

The object presented is to demonstrate one permanent monitoring device, which allows a study of the data obtained during the monitoring of the activity generated during resting time. This data will be interpreted depending on the final needs of the users.

### BACKGROUND OF THE INVENTION

At present there are various devices used for the monitoring of persons with some sort of handicap or those who do not have complete autonomy, for example people who are hospitalised, in retirement homes or children etc.

The vast majority of the different devices that are found on the market are controlled by presence, based on one element which is placed under the bed, which detects the presence or not of the user, based on the pressure changes which are registered by the data gathering elements.

Another type of device are those whose purpose is the detection of liquids or damp on the surface of the bed, focusing mainly on beds for children and the elderly.

The idea of the invention is to monitor the present movement on a surface of rest, assuming that all the results obtained by the different element can be analysed, stored and managed via a web interface or computer application.

The range of the application of the invention will be defined in function by the characteristics of the person responsible to study and observe the data obtained, they will be defined in three different areas:
- Leisure: If the data interpreted by the user for self monitoring.
- Control: If the data is interpreted by a third person in charge of controlling the surface where the device is located. In this case, the third person will manage the monitoring of the results.
- Professional/Healthcare: If the data is interpreted by a professional. In this case the monitoring will be mostly for healthcare purposes.

Regardless the range of the application, a history of the generated activity on the surface will be generated.

### DESCRIPTION OF THE INVENTION

The monitoring system activity on resting surfaces has the following technical features:
- Distribution of a network of elements capable of collecting variations in different parameters, integrated in a protective surface.
- The integration of the collecting elements shall be such that the end user is unaware of the collection. This means that both the surface on which the distributed network of collecting elements is and the network itself will therefore be invisible to the end user.
- Reading of collected data and processing them.
- Storing data either locally or remotely in a database.
- Sending the collected data to a database through different technologies, whether wired or wireless.
- Ability to access the database by the user.

The variations capture elements to be distributed at least on the surface defined are:
- At least one element capable of capturing the temperature change at the surface.
- At least one element capable of capturing the movement variation at the surface with respect to the three axes.
- At least one element capable of capturing the pressure variation over the surface pressure.
- At least one element capable of capturing the variation of surface moisture.
- The types of items that capture variations presented above may be extended. This extension may result in a number of collection elements and a variety of them.

For the data reading and analysis the system uses an integrated controller in the invention. The functional features of the device controller are as follows:
- Interpret and manage all data captured, whether analogue or digital measurements.
- Integration of the controller as a part of the surface, so that it is not apparent to the end user during use.
- Power supplied is either via batteries, or directly from the electric network.
- Managing the communication system to the server where the database is stored will be where the information will be located.
- This controller will be integrated into a plate on which the inputs of the variations data gathering elements, power and communication interface are all connected. The communication interface could be both wireless and wired.

Once performed all defined protocols to manage data gathering elements and the data are storage in the database, the user previously registered, is able to access them. This allows the user to have a history of recorded activity on the resting surface.

It is possible to store the data either locally or remotely. Each registered user will be linked to a particular device; a unique and characteristic parameter of every device will be assigned to each user, avoiding the multiplicity of the registers.

User registration is necessary to link the data stored in the database with each of the users. Depending on where the database is located this record will be local or remote.

### BRIEF DESCRIPTION OF THE DRAWINGS

Description of the figures.

Below is a description of the drawings being submitted.
Figure 1:

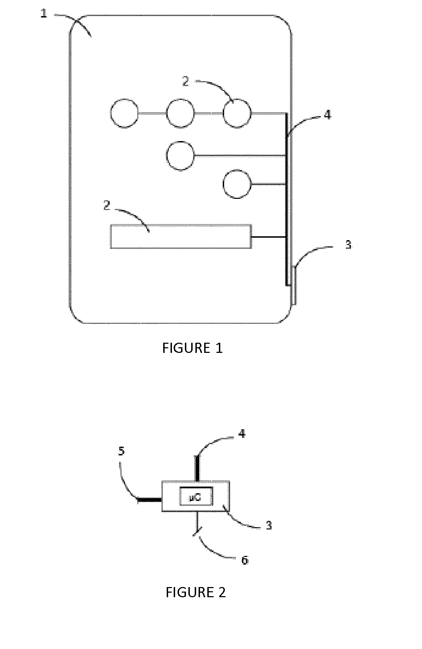

   In figure 1 we can observe a section of the plant layout of the invention. The different parts in this section are:
      - The surface integrating all the components (1).
      - An example of a possible configuration of the elements responsible for capture (2) variations in the parameters established as study parameters.
      - Place to locate the controller (3) and connections attached (Figure 2)
Figure No. 2:
   This figure shows the wiring of the controller. These connections are inputs (4) of all collecting elements which are distributed over the surface, input power to the device (5) and the output of the communication interface (6) to each one of the devices.

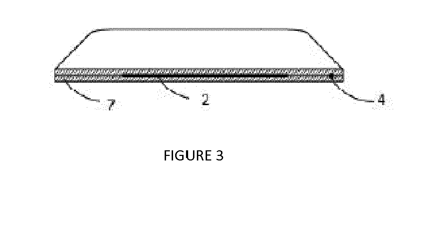

In figure 3 is shown a sectional conical view of the invention. In it can be found the following elements:
   - Place where the collecting elements are arranged (2).
   - Connection Bus (4) between each of the collecting elements (3) and the controller (3).
   - Material which will serve to cushion (7) and protect the collecting elements (2) placed on each faces of the invention.

### DESCRIPTION OF A PREFFERED EMBODIMENT

As can be seen in the figures described above, the device (1) is located on the sleeping/resting surface. This is where the activity is generated which will then be collected by the collecting elements (2). Said elements variations uptake is distributed over the surface so that each one captures variations or parameter measurements independently. These elements are directly connected to the controller (3) via a connection bus (4), the controller analyzes the data and processes them for subsequent delivery to the database via the communications interface (6).

Various devices are connected to the controller (3), within them the devices in charge of collecting variations (2), the interface (6) that enables the data traffic between the device and the database where the data is stored and finally the power supply input (5) of the device. Depending on the kind of interface used, the data could use a wired or wireless technology.

Once the data is received in the database, either locally or remote, they could be represented for the interpretation either through a web app or in managing software.

## Claims

1. RESTING SURFICE MONITORING SYSTEM, composed by different parts, first of them is the resting surface (1) wich integrates the distribution of sensor elements that form the invention, while another of the defined part is the one in charge of the managing of the logical data gathering from the elements (2), composed of an electronic circuit (3) in charge of the monitoring and managing of the variations produced by the sensors and allowing the information sending through different communication technologies, such as guided or not guided communication systems, with a data base thata allows the administration and managing of the storaged data.
